# EUROPÄISCHE PATENTANMELDUNG

(11) **EP 2 417 935 A2**
(43) Veröffentlichungstag der Anmeldung: **15.02.2012**
(21) Anmeldenummer: 11187798.1
(22) Anmeldetag: 07.06.2005
(51) Int. Cl.: A61F 2/28, A61L 27/56, A61L 27/44

(54) **Implantatkörper mit Wirkstoff**

(30) Priorität: 07.06.2004 DE 102004027657
(62) Teilanmeldung aus: 05756998.0
(71) Anmelder: ZOW Zentrum für orthopädische Wissenschaften GmbH, 81545 München (DE)
(72) Erfinder: Draenert, Klaus, 81545 München (DE)
(74) Vertreter: Vossius & Partner

(57) **Zusammenfassung**

Die Erfindung betrifft einen Implantatkörper (20), dessen Oberfläche mindestens teilweise durchbrochen ist von im Durchmesser einstellbaren Poren, mit einer inneren Struktur aus von Schalen (22) umgebenen Hohlräumen, wobei die Hohlräume ein Hohlraumsystem bilden, dessen Hohlräume im Durchmesser einstellbar und durchgehend interkonnektierend verbunden sind, wobei das Hohlraumsystem der inneren Struktur durch die Oberfläche des Implantatkörpers nach außen geöffnet ist, und wobei der Implantatkörper einen kurzzeitig oder protrahiert freisetzbaren Wirkstoff enthält.

## Beschreibung

In der Chirurgie am Bewegungsapparat besteht seit jeher ein Bedarf, Knochendefekte nach Frakturen, nach Entfernung von Tumoren, Knochensubstanzverluste nach Entzündungen oder in Verbindung mit Knochenzysten mit Knochenmaterial oder mit einem dem Knochen ähnlichen Ersatzmaterial wieder aufzufüllen. Hierbei wurde zum Teil auch auf bovines Ersatzmaterial zurückgegriffen [Tröster SD 1993: Die Hydroxylapatitkeramik Endobon®). Eine alternative Therapiemöglichkeit für Knochendefekte. In: Venbrocks R, Salis G von (Hrsg): Jahrbuch der Orthopädie,pp 231-246. Zülpich: Biermann] oder Material aus Korallenriffen [Irwin RB, Bernhard M, Biddinger A (2001) Coralline hydroxyapatite as bone substitute in orthopaedic oncology. Am J Orthop 30:544-550] im Labor aufbereitet (replaminiforme.Prozesse) und eingesetzt. Beiden Materialien haftet der Makel an, dass es biologische und damit gerichtete Strukturen sind, die man nicht mehr beeinflussen kann und die kaum standardisiert herstellbar sind. Beide Werkstoffe bringen die Anisotropie in Struktur und Eigenschaften einer gewachsenen biologischen Struktur mit und sind in fast allen Fällen zu steif. Ein Verfahren für die Herstellung solch biologischen Ersatzmaterials ist in der DE-A 3903695 beschrieben. Synthetische Ersatzwerkstoffe sind zwar aus reinen Ausgangsmaterialien herstellbar, lassen jedoch eine geordnete, dem Knochen ähnliche Struktur vermissen; ein solcher synthetischer Werkstoff ist das geschäumte Ceros®; diese Werkstoffe lassen einen knöchernen Durchwuchs vermissen, da die Poren meist geschlossen sind [Dingeldein E und H Wahlig (1992) Fluoreszenzmikroskopische Untersuchungen zur knöchernen Integration von Kalziumphosphatkeramiken. In: Merck Biomaterialien (Hrsg) Endobon® und DBCS®, Darmstadt:Merck].

In der Offenlegungsschrift DPA 2242 867 und US PA 38 99 556 wird ein Verfahren mit einem präformierten, formgebenden Gerüst beschrieben mit einer dichten Schüttung von Kugeln, bei dem die formgebenden Elemente von einem Lösungsmittel übergossen werden und auf diese Weise verklebt werden sollen; mit einem solchen Verfahren lässt sich jedoch kein gleichmässig poröses interkonnektierendes Material in stardardisierter Form herstellen; zu unregelmässig waren die Veridebungen und zu unkontrollierbar der Anlösungsprozess.

In der EP 0553167 und EP 0204786 wurde das Problem teilweise gelöst, indem deformierbare formgebende Elemente durch Druckbeaufschlagung in Kontakt aufeinander gepresst wurden und in diesem Zustand von der Gerüst-bildenden Masse umgossen wurde, welche danach aushärtete und anschliessend von den formgebenden Elementen chemisch oder thermisch wieder befreit wurden. Die so hergestellten Implantate liesen in der Hälfte, die der Druckbeaufschlagung zugewandt war, schöne und nahezu gleichmässige Interkonnektierungen erkennen, in der von dieser abgewandten Hälfte jedoch waren zahlreiche geschlossenen Poren ausgebildet. Formgebende deformierbare und nur teilweise elastischen Elemente dämpfen die einwirkende Kraft und die deformierende Wirkung erschöpft sich durch Dämpfung, sodass keine gleichmässige und durchgehende Interkonnexion erreicht wird. Rein zufällig wurde nun ein Verfahren gefunden, welches diese Phänomen vermeiden lässt und vollständig gleichmässige Verformung aller am Prozess beteiligten formgebenden Elemente erreichen lässt. Diese Verfahren ist denkbar einfach und daher auch besonders preiswert und vollständig reproduzierbar.

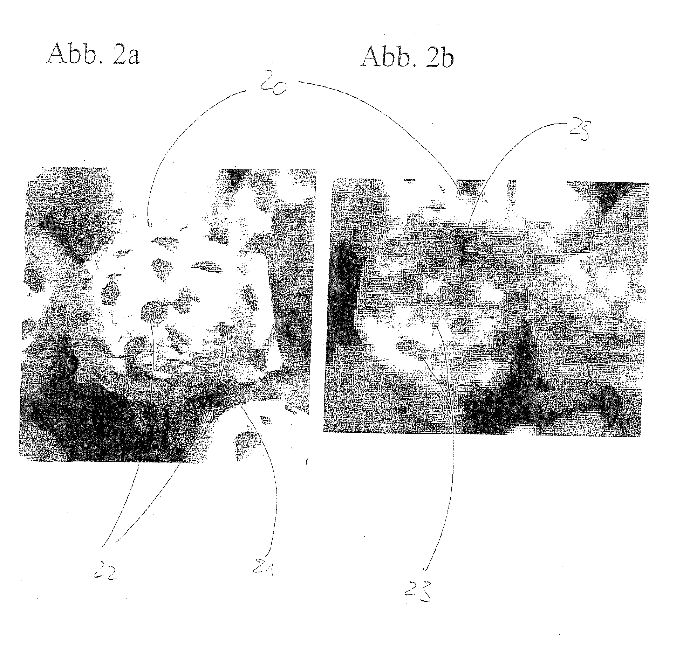

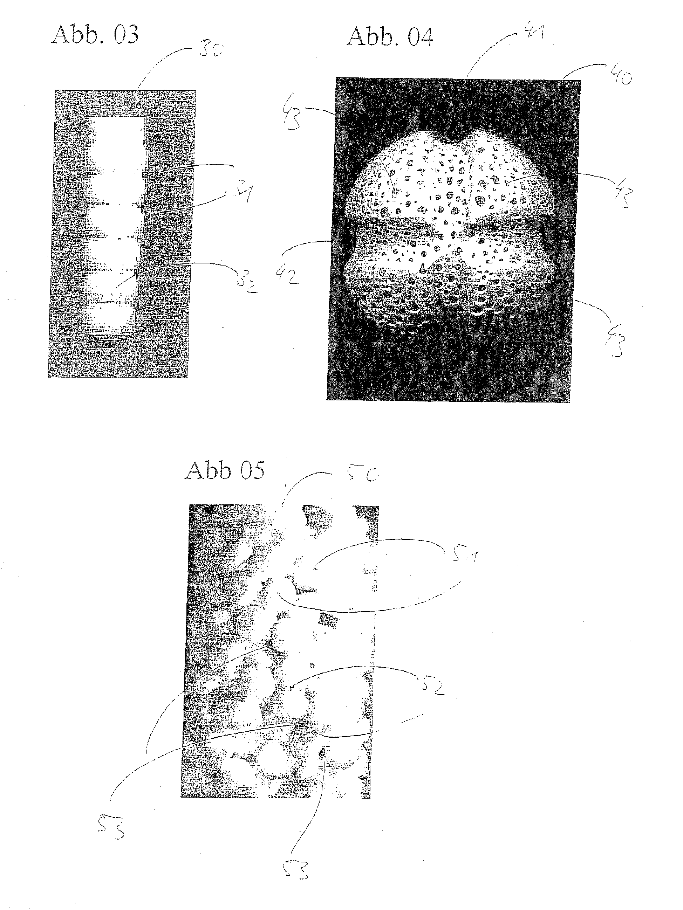

Die folgenden Punkte sind bevorzugte Ausführungsformen der vorliegenden Erfindung:
(1) Kugelförmiger, polymorpher oder auch andersförmiger, gekammerter Implantatkörper (Abb 2/20), bestehend aus einer vollständigen oder unvollständigen dicken oder dünnen Schale mit insgesamt, teilweise oder gernischt glatter, rauher oder strukturierter Oberfläche (Abb 2/21), über die Gesamt- oder Teilfläche durchbrochen von im Durchmesser einstellbaren runden oder rundlichen, gleichmässig oderwngleichmässig verteilten Poren, und einer inneren Struktur, bestehend aus einem Hohlraum oder einem Konglomerat aus von Kugelschalen (Abb 2/22) umgebenen Hohlräumen, die mit ihrem Schalengerüst in direktem Zusammenhang mit der äusseren Schale stehen, welches ein zusammenhängendes Hohlraumsystem aus hohlen Kugeln oder kugelförmigen Hohlräumen umschliesst, das über ein im Durchmesser einstellbares Porensystem im Schalengerüst durchgehend interkonnektierend verbunden ist und durch die äussere Schale nach aussen geöffnet ist.
(2) Implantatkörper nach Punkt (1) als Quader oder Würfel, Zylinder, Konus (Abb 03/30) oder Scheibe, Halbschale oder Halbkugel, Kugelsegment, Kugelschnitz, Keil oder Ring oder Scheibenring, Pyramide oder Pyramidenstumpf, Spirale oder Schraube oder jedwede andere Form.
(3) Implantatkörper nach den, **Punkten** (1) und (2) so beschaffen, dass die Aussenschale strukturiert ist in Form von zirkulären (Abb 03/31) oder radiären, longitudinalen, schrägen oder spiralförmigen, parallel oder sich kreuzend (Abb 04/41,42) verlaufenden Einschnürungen, Furchen oder Wülsten, oder beidem, einzeln oder in Vielzahl, oder in Form einzelner oder mehrerer Erhebungen, Eindellungen, Zapfen oder anderer Strukturen.
(4) Implantatkörper nach Punkt (1) - (3) so beschaffen, dass die innere Struktur einem schalenförmigen Schwamm- oder Spongiosaknochen entspricht (Abb 2b).
(5) Werkstoff oder Implantat nach **Punkt** (1) bis (4) so beschaffen, dass die Pore der äusseren Schale in ihrem Durchmesser kleiner ist als der Durchmesser des mit ihr in Verbindung stehenden Hohlraumes und zwar im Verhältnis 1:5 bis 1:1,5, im Mittel 1:2 bis 1:3 (Abb 2a, 2b und Abb 04/43).
(6) Implantatkörper nach den **Punkten** 1) bis (5), vorzugsweise in Form gekammerter Kugeln oder kugelförmiger Gebilde einer Grösse zwischen 0,8mm und 12mm im Durchmesser, im Mittel zwischen 1-2 mm und 3-6 mm im Durchmesser und einem interkonnektierend verbundenen inneren Porensystem mit Durchmessern der Poren zwischen 80µm und 1500µm, vorzugsweise 150um bis 600um (Abb 2a).
(7) Implantatkörper nach den **Punkten** (1)-(6) so beschaffen, dass ein Konglomerat von Einzelimplantaten ausgebildet ist, vorzugsweise aus kugelförmigen, die mit einstellbar breiten Brücken (Abb 05/52) miteinander verbunden sind und ein in sich zusammenhängendes Hohlraumsystem (Abb 05/53) umschliessen, das es erlaubt, dass die vorzugsweise kugelförmigen Implantatkörper in der Weise von Knochen umwachsen werden können, dass ein physiologisches Schwammknochengerüst entsteht, dessen Durchtritte durch die Verbindungsbrücken der Implantatkörper vorgegeben sind.
(8) Implantatkörper nach den **Punkten** (1) bis (7), so beschaffen, dass der Gerüstwerkstoff ein Ca-Phosphat, insbesondere Tri-Calciumphosphat oder Tetra-Calciumphosphat, ein Hydroxylapatit, eine Calcium-Carbonat oder Calcium -Sulfat-Verbindung ist, oder auch eine Alummiumoxyd-Keramik, ein Kollagen, eine Polyaminosäure oder ein anderes resorbierbares Polymeres, aber auch ein Acrylat oder Abkömmling davon, ein Polymethylmethacrylat oder ein Copolymer davon, mit oder ohne Calciumphosphat, Calcium-Sulfat oder Calcium -Carbonat als Füllwerkstoff, oder ein Metall, vorzugsweise eine CoCrMo-Legierung, Titan oder Tantal oder eine Legierung derselben ist.
(9) Implantatkörper nach den **Punkten** (1) bis (8) so beschaffen, dass das Hohlraumsystem ganz oder teilweise mit einem resorbierbaren oder nicht resorbierbaren organischen oder anorganischen Füllwerkstoff, mit dämpfenden oder versteifenden Eigenschaften, aufgefüllt oder beschichtet ist, und zwar in der Weise, dass die innere Füllung sich auf das Innere des Implantatkörpers beschränkt oder diesen vollständig ausfüllt, jedoch die äussere Schale nicht überragt, oder regelmässig oder unregelmässig aus den Poren der äusseren Schale hervorquillt und tropfenförmige und /oder kugelförmige oder ähnlich konfigurierte Erhebungen über die äussere Schale bildet, die gleichmässig oder ungleichmässig über die Oberfläche verteilt, Mikrobewegungen mehrerer gleicher oder ähnlicher Implantatkörper, beispielsweise in einer Schüttung, ganz oder nahezu ganz verhindern.
(10) Implantatkörper nach den **Punkten** (1) bis (9) so beschaffen, dass als Füllstoff ein resorbierbares Polymeres, beispielsweise eine Polyaminosäure, ein Polylactid, Polyglactin, Glycosid oder ähnlicher Werkstoff, ganz allgemein Polyacetale, Polysäureamide oder Polyester, aber auch nicht resorbierbare Polymere wie Polymethylmethacrylate oder ähnliche Polymere, Agarose oder ein Gemisch aus Agarose und einem Calciumphosphat oder Calciumkarbonat verwandt wird.
(11) Implantatkörper nach den **Punkten** (1) bis (10) so beschaffen, dass ausschliesslich das Zentrum oder die zentrale Achse oder die Hohlräume entlang der zentralen Achse, zusammenhängend oder einzelne Hohlräume verstreut oder Gruppen von Hohlräumen mit einem Werkstoff nach den . **Punkten** (9) und (10) aufgefüllt sind, der einen Wirkstoff enthält, der schnell oder langsam, kurzzeitig oder protrahiert freigesetzt wird und sich per Diffusion über die freie Implantatoberfläche zentrifugal verteilt.
(12) Implantatkörper nach den **Punkten .** (1) bis (11) so beschaffen, dass als Wirkstoff ein Wachstumsfaktor,ein Gewebshormon, und / oder ein Gemisch dieser Faktoren, beispielsweise als demineralisierte Knochenmatrix, oder ein Antibiotikum enthält oder ein Hormon, ein Immunsuppressivum, ein Zytostatikum oder ein Gemisch aus Wachstumsfaktoren und einem Antibiotikum oder eine Kombination aus verschiedenen Wirkstoffen, welche aus dem Trägerwerkstoff nach Punkt (11) in verschiedener Weise freigesetzt werden.
(13) Implantatkörper nach **Punkt** (12) so beschaffen, dass als Antibiotikum Gentamycin, Clindamycin, Streptomycin oder ein anderes bakteriostatisch oder baterizid wirkendes Mittel verwandt wird.
(14) Implantatkörper nach den **Punkten** (1)-(13) so beschaffen, dass in standardisierter Weise weite und engere Hohlraumsysteme ausgebildet sind, die zusammenhängend oder unterbrochen, einzeln oder in Gruppen oder auch entlang einer Achse angeordnet sind und unterschiedlich weite Interkonnexionen aufweisen und die Hohlräume sich damit leichter oder schwerer mit Füllwerkstoffen oder Wirkstoffen oder beidem ausfüllen lassen.
(15) Implantatkörper nach den **Punkten** (1) - (14) so beschaffen, dass verschieden grosse Hohlräume mit einem interkonnektierenden Gerüst aus einem anderen resorbierbaren oder nicht resorbierbaren Werkstoff aufgebaut sind, beispielsweise ein grosser Hohlraum mit einer schalenförmigen Wendung aus Hydroxylapatit mit einem schalenförmig aufgebauten Gerüst aus einem leicht resorbierbaren Tricalciumphosphat, sodass in dieser Weise ein durchgehend kombinierter, beispielsweise aus Hydroxylapatit und Tricalciumphosphat bestehender, oder umgekehrt oder ein aus anderen Werkstoffen kombinierter Implantatkörper entsteht.
(16) Verbundimplantatkörper, bestehend aus einem Implantatkörper I der **Punkte** (1) - (15), und einem Implantatkörper II dieser Ansprüche, jedoch mit sich unterscheidender Porosität, mit oder ohne Füllwerkstoff mit oder ohne Wirkstoff, teilweise oder vollständig in seinem Hohlraumsystem aufgefüllt, in der Weise verbunden, dass letzterer entlang einer zentralen Achse mechanisch eingesetzt ist, sei es, dass durch Schleifen oder eine andere Trennmethode ein zentraler Zylinder herausgenommen wurde oder dieser durch das Herstellverfahren des Implantatkörpers I bereits ausgespart ist.
(17) Verbundimplantatkörper, bestehend aus einem Implantatkörper I und einem mechanisch in diesen eingesetzten Implantatkörper II, welcher aus einem porösen oder soliden Werkstoff mit oder ohne Wirkstoff besteht und entweder resorbierbar oder nicht resorbierbar ist, beispielsweise aus einem Werkstoff des **Punktes** (9) - (12) besteht, mit oder ohne einem Wirkstoff, wie in den **Punkten** (11) und (12).
(18) Verfahren zur Herstellung von Implantaten nach den **Punkten** (1) bis (15) mit den folgenden Schritten:
   - Schüttung von formgebenden Elementen in eine Form mit perforiertem Boden und Deckel ohne dass formgebende Elemente herausfallen können, im einfachsten Falle mit einem Siebraster verschlossen, Boden und Deckel so zueinander angeordnet, dass die formgebenden Elemente in festem Kontakt gehalten werden können.
   - Intrusion der Form mit den in dieser Form gehaltenen, formgebenden Elementen in eine Passform, beispielsweise einen Becher, der die Form schlüssig aufnimmt, gefüllt mit heissem Wachs einer Schmelztemperatur zwischen 60°C und 90°C, in der Regel um 80°C, und Aushärten des Wachses; aber auch Aufgiessen oder Einsaugen von Wachs ist möglich.
   - Herausziehen der Form mit ausgehärtetem Wachsgerüst, welches die formgebenden Elemente umfasst, und Auslösen oder Entfernen der formgebenden Elemente, beispielsweise Auflösen von lufthaltigen Polystyrolkugeln als formgebende Elemente in Aceton oder Herauslösen von Zuckerkugeln im Wasser oder einem ähnlichen Entfernen anderer physikalisch oder chemisch wieder entfernbarer Formkörper, welches das Wachsgerüst nicht angreift;
   - Auffüllen des so entstandenen Hohlraumsystems mit kleineren formgebenden Elementen, beispielsweise Zuckerkugeln im Rieselverfahren mit Hilfe eines Rüttlers oder Styropor®-Kugeln im Einsaugverfahren mit einem Sauger und einem Siebdeckel, um das Durchsaugen der formgebenden Elementen zu verhindern;
   - Auffüllen des verbleibenden Hohlraumsystems um die formgebenden Elemente mit einem fliessfähigen Werkstoff, beispielsweise einem Gemisch aus Agar-Agar und einem Calciumphosphat, wie Tricalciumphosphat oder Hydroxylapatit oder auch einem anderen fliessfähigen und aushärtbaren Werkstoff, wie Polymethylmethacrylat(PMMA) oder einem Copolymeren davon oder auch einem anderen resorbierbaren oder nicht resorbieren fliessfähigen Werkstoff.
   - Festigen des fliessfähigen Werkstoffes um die formgebenden Elemente im Wachsgerüst, sei es durch Abkühlen des Agar/Calciumphosphatgemisches, sei es durch Selbstaushärten eines PMMA Werkstoffes und anschliessend Herauslösen der formgebenden Elemente und Entfernen des Wachsgerüstes durch ein hierfür geeignetes Verfahren, beispielsweise Herauslösen von Zuckerkugeln oder auskristallisierten Salzen aus einem PMMA-Gerüst in Wasser, oder Herauslösen von lufthaltigen Polystyrolkugeln aus einem Agar-Calclumpbosphatgemisch in Aceton und anschliessendes Herauslösen vom Wachs in einem geeigneten Lösungsmittel, beispielsweise Methylcellulosesolvacetat, oder einfach das Verbrennen des Wachses während des Sintems in einem Sinterofen bei Temperaturen zwischen 900°C und 1400°C,
(19) Verfahren nach. **Punkt** (18) mit den darin beschriebenen Verfahrensschritten, so durchgeführt, dass als formgebende Elemente Zucker-oder Salzkugeln zwischen 300µm und 15mm verwandt werden und diese in Wasser herausgelöst werden und die so entstanden Poren des Wachsgerüstes mit kleineren Zucker oder Salzkugeln, oder lufthaltigen Polystyrolkugeln oder anderen leicht löslichen lufthaltigen oder soliden Kunststofflcugeln aufgefüllt werden, die anschliessend von einem Gerüstwerkstoff umflossen werden und danach mit Wasser oder Lösungsmittel oder auch Hitze wieder entfernt werden.
(20) Verfahren nach **Punkt** (18) und (19) mit den darin beschriebenen Verfahrensschritten, ergänzt durch einen Verfahrensschritt:
   - lufthaltige formgebende Elemente in den Poren des Wachsgerüstes werden in einer temperierten vakuumdichten Kammer einem definierten Unterdruck ausgesetzt,und gleichzeitig über eine kühlbare Stellplatte abgekühlt, was dazu führt, dass sich die lufthaltigen formgebenden Elemente bei ihrer Ausdehnung das noch weiche Wachs in die Zwischenkugelräume verdrängen und sich breitere Kontaktbrücken zwischen den Elementen ausbilden, insbesondere auch an der Grenzzone zum Wachs; das Ergebnis ist nach Einsaugen des Gerüstwerkstoffes und dessen Aushärten eine porösere Struktur der Implantatkörper, inbesondere eine durch die Höhe und Dauer des einwirkenden Vakuums sehr exakt einstellbare und damit definierbare Interkonnexion und Oberflächenporosität.
(21) Verfahren nach **Punkt** (20) mit den in den **Punkten** (18) und (19) beschriebenen Verfahrensschritten, modifiziert im folgenden Verfahrensschritt:
   - lufthaltige formgebende Elemente in den Poren des Wachsgerüstes werden von einem fliessfähigen Gerüstwerkstoff umflossen und vollständig aufgefüllt; der Implantatkörper wird anschliessend mit den Luft enthaltenden, meistens geschäumten, formgebenden Elementen und dem diese umgebenden Gerüstwerkstoff in eine, beispielsweise über die Stellplatte temperierbare Vakuumkammer gestellt und bei 40-60°C, vorzugsweise 50°C , mit einem definierten Unterdruck beaufschlagt; dabei kann es auch vorteilhaft sein bei gekühlter Stellplatte bei 4°C bis 12°C und erhöhtem Unterdruck zu arbeiten; dieser führt zur Ausdehnung der formgebenden Elemente und beeinflusst daher direkt die Makroposität und die Durchtritte (Interkonnexionen), vor allem aber auch die Oberflächenporen; das Vakuum ist zwischen 800 mbar und 150 mbar Absolutdruck exakt und über eine Schaltuhr auch zeitlich einstellbar, die Temperatur in dem Gefäss ist schnell abkühlbar, z.B.über eine Stellplatte aus Metall, sodass das erreichte Ergebnis durch das Steifwerden des Wachsgerüstes festgehalten wird.
(22) Verfahren nach . **Punkt** (18) mit den folgenden Modifikationen:
   - Schüttung von formgebenden Elementen, beispielsweise Zuckerkugeln, in ein Werkzeug und Umspritzen der Kugelschüttung mit Polystyrolschaum, welcher danach aushärtet.
   - gegebenenfalls Zwischenlagerung in einer definiert feuchten Kammer, ein Vorgang, der zum Anlösen der Zuckerkugeln im Interface zum Gerüstwerkstoff führt und zu Fliessbrücken zwischen den formgebenden Elementen, ein Vorgang, welcher durch regelmässiges Umlagern der Implantatkörper gleichmässig über das Implantat verteilt werden kann, danach Trocknen im Trockenofen und Entfernen der Formkörper mit Wasser oder Hitze, ohne den Schritt einer Zwischenlagerung in einer feuchten Kammer erfolgt das Entfernen der formgebenden Zucker- oder auch Salzelemente, beispielsweis als Kugeln, im Wasser sofort.
   - Auffüllen der Hohlräume im geschäumten Polystyrolgerüst mit formgebenden Elementen und Fortfahren wie in den **Punkten** (18) bis (21) beschrieben.
(23) Verfahren nach **Punkt** (18) mit der folgenden Modifikation:
   - Als Gerüstwerkstoff wird giessfähiges und selbsthärtendes Silikon verwandt, welches Zuckerkugeln oder auch lufthaltiges Polystyrol als formgebende Elemente, oder solide Metall-oder Plastikkugeln oder Formelemente, welche anschliessend chemisch, physikalisch oder auch vorsichtig mechanisch wieder entfernt werden können, beispielsweise zu 4/5 umfliesst
   - das Auffüllen der Poren des Silikongerüstes mit kleineren formgebenden Elementen kann beispielsweise mit Zuckerkugeln, aber auch mit geschäumten Polystyrolkugeln erfolgen,welches im ersteren Fall im Rieselverfahren und im zweiteren vorzugsweise durch Einsaugen über ein Siebgitter erfolgt.
   - Wird im Anschluss daran das Poren und Spaltensystem wie unter **Punkt** (18) beschrieben, beispielsweise mit einem Agar/Hydroxylapatit Gemisch aufgefüllt und anschliessend in einer Vakuumkammer einem definierten Unterdruck ausgesetzt, so bilden sich Durchtritte und Randporositäten aus, aufgrund der Deformierbarkeit des Werkzeugs und der Expansion der formgebenden Elemente.
(24) Verfahren zur Herstellung eines Implantatkörpers nach den Punkten (1) bis (17) mit folgenden Verfahrensschritten:
   - die äussere Form des Implantatkörpers wird mit seinen definitiven Massen mit dem Schrumpfungsfaktor des Werkstoffes des Stützgerüstes multipliziert und mechanisch oder im Abdruckverfahren oder auch über CAD/CAM hergestellt.
   - mit dieser Form wird in einem Werkzeug mit Silikon im Abgussverfahren eine Silikonform hergestellt, die bis auf den Deckel den Implantatkörper wiedergibt,
   - das Silikonwerkzeug wird mit den formgebenden Elementen gefüllt und mit einem Silikondeckel verschlossen; dieser enthält eine Öffnung zum Befüllen im Deckel und eine oder mehrere Öffnungen zum Belüften am Boden oder umgekehrt;
   - über den Deckel wird das Werkzeug mit der plastischen Masse des Stützgerüstes, beispielsweise einem Gemisch aus Agar-Agar und Hydroxylapatit, befüllt;
   - anschliessend wird das Werkzeug mit den formgebenden Elementen, umfasst von der Masse des Stützgerüstes in einer geschlossenen Kammer mit einem Unterdruck beaufschlagt und abgekühlt.
   - es folgt die Herausnahme aus der Form und das Entfernen der formgebenden Elemente, beispielsweise der geschäumten Polystyrolkugein in Aceton.
   - nach der schrittweisen Dehydratation im Aceton wird der Implantatkörper in der Kühltruhe langsam an Luft getrocknet und anschliessend im Exsikkator unter Einwirkung von Phosphorpentoxyd weiter dehydratisiert.
   - der so getrocknete Implantatkörper aus beispielsweise einem Agar-Agar/Hydroxylapatit oder auch Agar-Agar/TCP Gemisch wird im Sinterofen gebrannt; der so gewonnene gekammerte Implantatkörper zeigt eine durchgehende Porosität unter Einschluss seiner gesamten Oberfläche und ist masshaltig und frei von Rissen.
(25) Verfahren nach **Punkt** (24) zur Herstellung von gehämmerten kugelförmigen Implantatkörpern nach den **Punkten** (1)-(17) mit folgenden Modifikationen:
   - der Silikonabdruck wird von einem Monolayer von Stahl- oder Polyamidkugeln hergestellt, welche zu 4/5 umgossen werden und anschliessend aus dem elastischen Material mechanisch wieder entfernt werden.
   - das so entstandene Hohlraumsystem wird in einem Abfüllwerkzeug mit perforiertem Silikonboden mit kleineren formgebenden Elementen, beispielsweise geschäumten Polystyrolkugeln aufgefüllt und anschliessend mit einem perforierten Deckel mit perforierter Silikoneinlage abgeschlossen; Boden und Decke weisen Bohrungen für die Auffüllung mit der Gerüstmasse bzw. für die Entlüftung auf.
   - das Werkzeug wird gefüllt mit der Masse des Stützgerüstes, beispielsweise einem Gemisch aus Agar-Agar und HA oder Tricalciumphosphat, anschliessend in einer geschlossenen Kammer mit Unterdruck beaufschlagt und abgekühlt.
   - die so erstarrten Implantatkörper werden vorsichtig aus der Form gedrückt und es werden die formgebenden Elemente entfernt, beispielsweise die geschäumten Polystyrolkugeln in Aceton, dehydratisiert und anschliessend bei 1000°C - 1300°C gebrannt, beispielsweise in einem Zirconiumtiegel.
(26) Herstellen eines Implantates nach den **Punkten** (1) - (17), als Ergebnis der folgenden Verfahrensschritte:
   - Erstellen eines elastisch oder plastisch deformierbaren Werkzeuges, beispielsweise im Spritzgussverfahren, nach einer CAD/CAM Konstruktion oder einer anderen technischen Konstruktion, oder im Abdruck oder Giessverfahren von einer direkten Vorlage, in jedem Fall aber unter Berücksichtigung des Schrumpfungsfaktors und so beschaffen, dass es mit formgebenden Elementen befüllt werden und wieder verschlossen werden kann, beispielsweise als Sandwich oder als fast geschlossener Körper mit Öffnung und Deckel und Belüftungsvorrichtungen auf der Gegenseite;
   - Befüllen des deformierbaren Werkzeuges mit formgebenden Elementen, beispielsweise mit EPS-Kugeln (Expandable-Poly-Styrol) oder direkt Styropor®-Kugeln, oder anderen leicht wieder entfernbaren formgebenden Elemente in Kugel-oder auch anderer Form, beispielsweise Zucker-oder Salzkugeln, Gelatine oder andere leicht entfernbare Materialien;
   - Einbringen in ein Füllwerkzeug, beispielsweise ein Mutterwerkzeug in einer Spritzgussmaschine, oder einer anderen Abfüllvorrichtung für den Gerüstwerkstoff;
   - Auffüllen durch Einspritzen oder Einsaugen des Gerüstwerkstoffes mit oder ohne definierten Unterdruck;
   - Abkühlen des Werkzeuges, zusammen mit dem Implantat, in der Vakuumkammer unter definiertem Unterdruck und über eine definierte Zeit;
   - Herausnehmen des Implantates, zusammen mit den formgebenden Elementen und Auslösen der formgebenden Elemente physikalisch oder chemisch;
   - Brennen eines beispielsweise keramischen Implantates bei 900° C bis 1400° C im Sinterofen.

### Beschreibung der Erfindung

Die Anwendung von einem auf die formgebenden Elemente einwirkenden Unterdruck führte überraschend zu einem perfekten Ergebnis. Dabei werden die locker in ein Werkzeug überraschend zu einem perfekten Ergebnis. Dabei werden die locker in ein Werkeug geschütteten formgebenden Elemente entweder vor der Abfüllung in einem vakuumdjchten System mit einem definierten unterdruck beaufschlagt und anschliessend die gerüstbildende Masse eingesaugt und unter Einwirkung des definierten Unterdruckes bei definierter Temperatur über eine definierte Zeit aushärten gelassen, oder nach der Abfüllung, zusammen mit dem Gerüstwerkstoff, mit definiertem Unterdruck beaufschlagt und gleichzeitig abgekühlt, beispielsweise über eine metallene Stellplatte:

Diese fast noch einfachere Anordnung führte aus dem Grunde zu weiter standardisierbaren Ergebnissen und beinhaltet folgende Schritte: die lockere Schüttung von formgebenden Elementen wurde mit der strukturbildenden Gerüstmasse umgossen und das geschlossene Gefäss, welches nicht vakuum - versiegelt war, wurde in einem dichten mit Unterdruck beaufschlagten Gefäss über eine bestimmte Zeit bei definierter Temperatur einem definierten Unterdruck ausgesetzt und gleichzeitig über die Stellplatte abgekühlt. Das einfache Handling dieses Vorgehens liess das Ergebnis hoch reproduzierbar werden.

Doch auch dieses Ergebnis konnte zufällig durch eine zunächst unscheinbare Veränderung, vor allem im Hinblick auf die einstellbare Porosität der Oberfläche, weiter verbessert werden: Nahm man nämlich statt eines festen metallischen Werkzeuges eine deformierbare Silikonform, so führte dies dazu, dass das Implantat, abhängig vom angelegten Unterdruck eine durchgehende bis in die Oberfläche reichende Porosität aufwies, die durch die Höhe des Unterdruckes auf der einen Seite und den E-Modul des Werkzeuges auf der anderen Seite einstellbar war. Dieses Verfahren liess auch dann eine durchgehende Porosität erreichen, wenn die formgebenden Elemente nicht expansionsfähig, also beispielsweise nicht lufthaltig, sondern beispielsweise Zuckerkugeln waren.

Als formgebende Elemente werden bei diesem Verfahren vorzugsweise expandierbare Polystyrol-Kugeln (EPS) verwendet, beispielsweise StyroporⓇ F414 das mit Pentan als Treibmittel aufgeschäumt ist. Beim Anlegen eines Unterdruckes dehnen sich diese Kugeln sehr schnell aus und nehmen an Volumen zu. Auf diese Weise wird die Kontaktbrücke zwischen den Kugeln breiter und bestimmt damit die Durchmesser der interkonnektierenden Durchtritte bis hinein in die Oberfläche; bei Verwendung eines Silikonwerkzeuges drücken sich die Kugeln in die Silikonwand und zudem zieht der Unterdruck die deformierbare Wand über die Kugeloberfläche in das Implantat hinein.

Geschäumte Werkstoffe zur Verwendung als formgebende Elemente werden bevorzugt eingesetzt, auch solche, denen Treibmittel zugesetzt sind, welche bei einer bestimmten Temperatur oder unter bestimmten Voraussetzungen aktiviert werden. Ein besonders bevorzugtes Material stellt das StyroporⓇ F414 dar mit bevorzugten Raumgewichten des aufgeschäumten Polystyrols zwischen 17g/l und 70g/l, vorzugsweise etwa 20g/l bis 35g/l. Die Komgrössenverteilung des aufgeschäumten Materials liegt zwischen 200µm und 15mm, häufig verwandt sind die Grössen zwischen 1000µm und 3000µm.

Zur Bestimmung der Expansion und der Deformierbarkeit der einzelnen formgebenden Elemente wurden Versuche durchgeführt, um in einfacher Weise die Parameter zu bestimmen, die zur Standardisierung des Verfahrens notwendig sind. Hierfür wurden verschiedene formgebende Elemente mit verschiedenen Raumgewichten, beispielsweise verschieden geschäumte Polystyrol-Kugeln mit verschiedenen Durchmessern, in einem Zylinder mit beweglichen, vakuumdicht anliegenden Kolben bis zu einer definierten Höhe von 84,3mm aufgeschüttet und einem definierten Vakuum ausgesetzt ; aus.der Veränderung der Ausgangshöhe in Abhängigkeit vom angelegten Unterdruck wurden Grössen ermittelt, die eine anfängliche Volumenreduktion durch Entfernen der Luft zwischen den formgebenden Elementen widerspiegein, gefolgt von einer Volumenausdehnung, die durch Aufbringen einer Kraft F, gemessen in N, zur alten Ausgangslänge einstellbar war und den Expansionsdruck der Luft in den formgebenden Elementen widergab. In Abhängigkeit von der Zeit nahm die Kraft langsam ab, was durch das Platzen der Luftblasen im Kunststoff zu erklären war. Aus diesem Phänomen wurden die definierten Zeiten ermittelt für die Beaufschlagung mit Unterdruck.

Drücke zwischen 150mbar und 800mbar, vorzugsweise um 300mbar bis 500mbar über eine Zeit von 15 Minuten einwirkend auf ein Calciumphosphat - Agar-Agar Gemisch, bei einer Temperatur des Implantates von 4-12° C zeigten besonders günstige Ergebnisse in Bezug auf die Aussenporosität und die inneren Interkonnexionen.

Als deformierbare Formen eignen sich vor allem giess- oder im Spritzgussverfahren verwendete Silicone einer Shorehärte unter 25 shore, vorzugsweise unter 18-20 shore. Es können aber alle plastisch oder elastisch deformierbaren Werkstoffe Verwendung finden, deren E-Modul deutlich unter dem der formgebenden Elemente liegt. Entsprechend können die Werkzeuge gegossen werden, aber auch in grossen Serien im Spritzgussverfahren hergestellt werden. Beispiele für plastisch deformierbare Werkzeuge sind Werkzeuge aus Styropor® verschiedener Dichte, Beispiele für elastisch deformierbare Werkzeuge sind die oben erwähnten Silikone, wobei auch geschäumte Silikone zur Anwendung kommen können. Der Expansionsdruck dieser Werkstoffe im Vakuum addiert sich in seiner Wirkung zu dem der formgebenden expansionsfähigen Elemente.

Als giessfähiges Material für den Gerüstwerkstoff wird vorzgsweise eine Mischung aus Hydroxylapatit (HA) oder Tricalciumphosphat (TCP) und einer Agar-Agar Lösung im Verhältnis 10g Pulver / 7ml bis zu 25ml Lösung, was einem Verhältnis von 1,4 bis 0,4 entspricht, ganz optimal sind Ansätze, bei denen das Mischungsverhältnis Pulver zu Lösung 0,45 bis 0,48 entspricht, beispielsweise 1600g HA auf 3500ml Lösung.

Je nach Zusammensetzung kann aus dem Ansatz bereits der Schrumpfungsfaktor berechnet werden: dieser liegt bei einem giessfähigen HA-Agar-Agar Gemisch, welches bei 60°C abgefüllt wird zwischen 0,95 bis 2,9, vorzugsweise zwischen 1,75 und 2,15, bei einem Verhältnis von 16g/35ml einer 1,7%igen Agar-Agar Lösung bei exakt 1,91. Mit diesen beiden Voraussetzungen, expandierbare formgebende Elemente, einer deformierbaren Silikonform und einem exakten Ansatz mit definierter Schrumpfung liessen sich sehr präzise Implantate "net shaped" sintern ohne, dass eine Nachbearbeitung notwendig wurde.

Das definitive Design multipliziert mit dem Schrumpfungsfaktor ergibt beispielsweise ein Implantatkörper in Kunststoff oder einem anderen im CAD/CAM Verfahren leicht bearbeitbaren Material, welches in einem Stammform bis zur Oberkante mit einem giessfähigen Silikon umgossen wird. Nach Aushärten des Silikons kann der formgebende Körper leicht wieder mechanisch entfern werden, die Silikonform wird am Boden mehrfach perforiert und danach mit Polystysrolkugeln der gewünschte Grösse aufgeschüttet, danach mit einem Silikondeckel mit Entlüftungslöchern verschlossen und in einer Abfüllform mit beispielsweise der Keramikmasse abgefüllt. Unmittelbar nach der Abfüllung wird das Werkzeug in toto in einem Exsiccator mit einem Unterdruck beaufschlagt und auf 4-12°C heruntergekühlt beispielsweise über die Stellplatte. Nach Aushärten der Keramikmasse kann das Werkzeug demontiert werden und der Grünling ist leicht zu entfernen. Im Acetonbad wird nun aus dem Keramik-StyroporⓇ-Implantat das Styropor herausgelöst, die Keramik dehydratisiert in Schritten 70/80/90 und 100%iges Aceton und anschliessend unter Kühlung schrittweise an Luft getrocknet (Kältetrocknung). Das Ergebnis wird stundenweise mit Hilfe einer Feinwaage dokumentiert, sowie kein weiterer Gewichtsverlust an Luft sich abzeichnet und die Gewichtskurve linear gleich bleibt, wird das Implantat über 24h im Exsiccator unter Zusatz von P₂0₅ unter Vakuum 150- 250 mbar Absolutdruck getrocknet und anschliessend bei 1300°C im Sinterofen gebrannt. Das Ergebnis ist ein offenporiges, absout masshaltiges Implantat mit einer gegenüber mechanisch nachbearbeiteten Knochenersatzwerkstoff-Zylindern oder Würfeln (Draenert et.al.2001 :Synthetische Knochenersatzwerkstoffe auf HA- und TCP-Basis Trauma Berufskrh 3:293-300 Heidelberg New York Berlin Tokyo: Springer) deutlich höhere Festigkeit, die durch äussere Strukturierung weiter vergrössert werden kann, beispielsweis Ringe, Einschnürungen, massive Kanten usw.

### Beispiel (1)

Styropor®-Kugel von 12mm Durchmesser werden in einem zylindrischen Gefäss mit perforiertem Boden mit Bohrungen von 10-11mm im Durchmesser und einem schlüssig den Kugeln aufsitzenden fixierbaren Deckel mit denselben Bohrungen in einen Becher getaucht und intrudiert, der mit 90°C heissem Wachs gefüllt und hierfür aus dem Heizofen genommen wird. Der Behälter mit den Styropor®-Kugeln passt dabei mit seinem Aussendurchmesser schlüssig in das zylindrische Wachsgefäss, welches einen demontierbaren Boden aufweist.

Nach Aushärten des Wachses bei Zimmertemperatur, wird der Boden des Wachsgefässes demontiert und der Styropor®-Behälter herausgedrückt. Der Styropor®-Behälter wird ebenfalls von seinem Boden und dem Deckel befreit und der Wachs-Styropor®-Zylinder herausgeschoben und in einem Acetonbad von dem Styropor® befreit. Nach Trocknung an Luft wird das so entstandene durchgehend interkonnektierend poröse Wachsgerüst in eine dieses schlüssig aufnehmendes Abfüllgefäss eingeschoben. Das Abfüllgefäss weißt einen Einfüllstutzen mit Perforationen am Boden des Gefässes auf und kann ausserdem ein Sieb aufnehmen, welches zum Einsaugen kleinerer Styropor®-Kugeln benötigt wird, ausserdem ist es mit einem Deckel versehen, welcher Entlüftungsbohrungen aufweist, beispielsweise mit einem Durchmesser von 1,5 - 2,5mm. In das Wachsgerüst werden Styropor®-Kugeln mit einem Durchmesser von beispielsweise 600-1200µm eingesaugt, wobei im Abfüllstützen ein Sieb mit einer Maschenweit von 400µm vorgeschalten wird, Das Hohlraumsystem des Wachsgerüstes füllt sich vollständig mit den kleineren Styropor®-Kugeln auf und wird danach mit einem Deckel verschlossen. Das Gefüss wird nun mit einer Keramikmasse abgefüllt, wobei über den Abfüllstutzen die Masse über die Belüftungslöcher eingesaugt oder ohne grossen Druck einfach über den Stutzen aufgefüllt wird. Tritt die Keramikmasse aus den Entlüftungslöchern des Deckels aus, ist die Form gefüllt und wird nun mitsamt dem Werkzeug in einen Exsiccator gestellt und über 15 Minuten mit einem Unterdruck von 500-600mbar beaufschlagt und während dieser Zeit über die metallene Stellplatte abgekühlt. Der so ausgehärtete Wachs-Keramik-Styropor®-Block wird anschliessend im Acetonbad vom Styropor® befreit, an Luft getrocknet und mit dem Wachs im Ofen bei 1300°^{c} gesintert. Das Ergebnis sind vollständig isolierte, aussen durchgehend poröse und im Innern vollstandig interkonnektierend gekammerte Kugeln mit einem Durchmesser um 6mm. Aufgrund der Schrumpfung des Matrixmaterial kommt es zur Ausbildung vollständig separierter Kugeln.

### Beispiel (2)

Statt einzelner gekammerter Kugeln kann ein interessanter Werkstoff gefertigt werden, der einem Negativabdruck der Knochenmarkswaben entspricht und ein Kugelkonglomerat mit Zwischenräumen zwischen den gekammerten Kugeln für das Einwachsen von Knochenbälkchen und breiten Verbindungsbrücken, die den Kontaktstellen der Kugeln entsprechen: Die Schritte entsprechen dem Beispiel 1, jedoch wird das Wachs-Styropor®-Gerüst während des Aushärtens in einem Exsiccator einem definierten Unterdruck von beispielsweise 600mbar ausgesetzt. Die daraufhin erfolgende Expansion der Styropor®-Kugeln führt zu verbreiterten Brücken und dickeren. Verbindungsarmen zwischen den Kugeln. Nach Aushärten des Wachs-Styropor®-Gerüstes erfolgt das Herauslösen des Styropor®. Die nachfolgenden Schritte entsprechen dem Beispiel 1 mit Einsaugen von kleineren Styropor®-Kugeln, Auffüllen mit einer Keramikmasse, Nachevakuieren unter Abkühlen und anschliessendem Herauslösen der formgebenden Elemente im Aceton; danach erfolgt im Gegensatz zum Beispiel 1 der Kälte-Trocknungsschritt: in Schritten von jeweils 2h wird über 70/80/90 und 3x.100 %iges Aceton in Stufen in der Kühltruhe an Luft getrocknet, bei 4-12°C aufsteigend in beispielsweise 4 Schritten von jeweils 3 Stunden bis zur Zimmertemperatur und anschliessender Dehydratation im Exsiccator unter Zuhilfenahme von P₂0₅ und einem Vakuum um 150mbar Absolutdruck über 24h. Bei einem solchen Vorgehen bleiben breite Brücken zwischen den gekammerten Kugeln bestehen und das Wachsgerüst kann mitsamt dem Keramikinlet im Ofen gebrannt werden bei beispielsweise 1300°C; das Wachs kann auch vorher im Heizofen abgeschmolzen werden bei 90°C, Voraussetzug hierfür ist, dass die Keramikmasse bereits luftgetrocknet ist. Das Ergebnis ist ein perfektes Gerüst aus zusammenhängenden im Inneren perfekt interkonnektierend gekammerten Kugeln mit durchgehend poröser Oberfläche und einer erstaunlich hohen Kompressionsfestigkeit. Diese liegt je nach Kugelgrösse bei 4-12Mpa.

### Beispiel 3

Die Herstellung eines masshaltigen Würfels mit einer Kantenlänge von 15mm und einer offenen Porosität über alle Flächen: Es wird bei einer Matrixmasse von 16/35 HA/-Agar-Agar Suspension 1,7%ig ein Schrumpfungsfaktor von 1,91 berechnet und im CAD CAM Verfahren ein Würfel aus POM mit einer Kantenlänge von 28,65mm hergestellt. Da der Würfel eine hohe Festigkeit von 4-6 Mpa erreichen soll, werden die Kanten nicht abgerundet. Der Würfel wird in ein Formwerkzeug gestellt und bis zu seiner Oberkante mit einem selbsthärtenden Silikon ausgegossen. Nach 24h wird der Würfel mechanisch entfernt und das Silikonwerkzeug in ein Abfüllwerkzeug eingebracht, dessen Boden innen aus einem perforierten Silikonboden besteht und nach Auffüllen mit 1200µm messenden Styropor®-Kugeln mit einem Silikondeckel mit Entlüftungslöchern schlüssig abgedeckt. Nachdem das Werkzeug mit einem Schraubdeckel mit Entlüftungslöchern verschlossen wurde, wird es mit einer Keramimasse aufgefüllt; anschliesend erfolgt im Exsiccator auf einer metallenen und kühlbaren Stellplatte die Beaufschlagung mit Unterdruck von 500mbar über 15 Minuten bei gleichzeitiger Abkühlung. Danach wird das Werkzeug demontiert und der Keramik-Styropor®-Würfel vorsichtig mechanisch herausgenommen und im Acetonbad von den formgebenden Elementen befreit. Zur rissfreien Trocknung wird die Kältedehydratisierung angeschlossen wie in den Beispielen 1 und 2 beschrieben und anschliessend der Würfel im Ofen bei beispielsweise 1300°C gesintert. Das Ergebnis ist ein masshaltiger sehr kompressionsstabiler Würfel mit offener Porosität über alle Flächen und einer durchgehend interkonnektierenden Porosität der inneren Struktur und verstärkten Kanten aus kompakter Keramik.

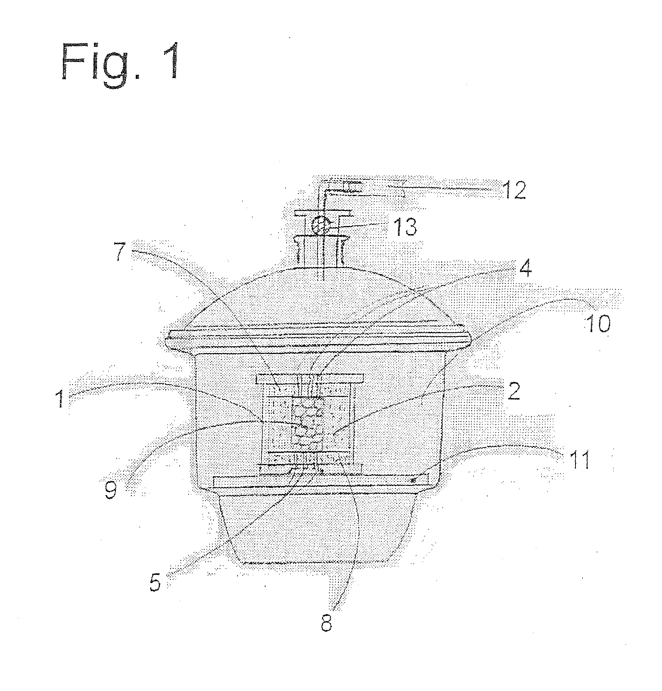

in der Abbildung 1 ist eine einfache Vakuumkammer beschrieben in Form eines Exsiccators (10) mit Belüftungsventil (13) und Hahn zum Anschluss eines Schlauches zur Vakuumpumpe (12) und einer metallenen Kühlplatte (11) mit einem auf der Kühlplatte stehenden Abfüllwerkzeug (1) mit einem schlüssig aufgenommenen deformierbaren Werkzeug in drei Teilen, einen Korpus (2), einen Deckel (7) mit Perforationen (4) und einem Boden (8) mit Perforationen (5) zum Auffüllen beispielsweise mit einer keramischen Masse.

In der Abbildung 2a und 2b sind keramische Implantate des Anspruchs 1 dargestellt (20), einmal in der Aufsicht und in der Abbildung 2b als Bruch. Die Aufsicht auf die Kugelschale lässt die offene Porosität (22) erkennen und die rauhe Schale (21). Im Bruch ist die interkonnektierende Porenstruktur (23) dargestellt.

In der Abbildung 3 ist ein Implantat aus den Ansprüchen (I) - (3) dargestellt als Knochendübel (30), beispielsweise zur Refixation eines Bandes beim Kreuzbandersatz über die Aussenseite des Dübels, welches horizontale (31) Einschnürungen aufweist, die vorteilhaft jedoch auch schraubenförmig angeordnet sein können, und welches über die gesamte Oberfläche eine von Poren (32) unterbrochene äussere Schale aufweist.

In der Abbildung 4 ist ein leichtes Keramikimplantat (40), net shaped gesintert, mit einer offenen Porosität über die gesamte Oberfläche (43), welches sich kreuzende Furchen oder Rinnen (41,42) aufweist, in denen die Augenmuskeln vernäht werden können, welches als Teil eines künstlichen Auges verwendet wird.

Die Abbildung 5 zeigt ein Kugeikonglomerat (50)_{;} welches aus soliden Kugeln (51) besteht, welchesjedoch auch aus Elementen der Abbildung 2 bestehen kann und sich äurcl7 breite Brücken (52) zwischen den Kugelelementen und regelmässigen Zwischenkugelräumen (53) auszeichnet.

## Patentansprüche

1. Implantatkörper (20), dessen Oberfläche mindestens teilweise durchbrochen ist von im Durchmesser einstellbaren Poren, mit einer inneren Struktur aus von Schalen (22) umgebenen Hohlräumen, wobei die Hohlräume ein Hohlraumsystem bilden, dessen Hohlräume im Durchmesser einstellbar und durchgehend interkonnektierend verbunden sind, wobei das Hohlraumsystem der inneren Struktur durch die Oberfläche des Implantatkörpers nach außen geöffnet ist, und wobei der Implantatkörper einen kurzzeitig oder protrahiert freisetzbaren Wirkstoff enthält.

2. Implantatkörper nach Anspruch 1, wobei die Oberfläche eine äussere Schale ausbildet, die über ihre Gesamt- oder Teilfläche durchbrochen ist von den Poren, und wobei die Hohlräume der inneren Struktur von Schalen (22) umgeben sind, wobei die Schalen ein Schalengerüst bilden, dass in direktem Zusammenhang mit der äusseren Schale steht, und wobei das Hohlraumsystem der inneren Struktur durch die äussere Schale nach außen geöffnet ist.

3. Implantatkörper nach Anspruch 1 oder 2, wobei die Hohlräume zumindest teilweise mit einem Füllwerkstoff aufgefüllt sind, der den Wirkstoff enthält.

4. Implantatkörper nach Anspruch 3, wobei die Hohlräume im Zentrum oder entlang einer zentralen Achse des Implantatkörpers zusammenhängend oder nur einzelne Hohlräume oder Gruppen dieser Hohlräume mit dem Füllwerkstoff aufgefüllt sind.

5. Implantat nach einem der Ansprüche 1 bis 4, wobei der Wirkstoff per Diffusion über die freie Oberfläche des Implantatkörpers zentrifugal verteilbar ist.

6. Implantatkörper nach einem der Ansprüche 1 bis 5, wobei der Wirkstoff ein Antibiotikum enthält.

7. Implantatkörper nach Anspruch 6, wobei als Antibiotikum Gentamycin, Clindamyin, Streptomycin oder ein anderes bakteriostatisch oder bakterizid wirkendes Mittel verwandt wird.

8. Implantat nach einem der Ansprüche 1-7, wobei der Implantatkörper einen Wachstumsfaktor, ein Gewebshormon, ein Hormon, ein Immunsuppressivum, ein Zytostatikum oder eine Kombination aus verschiedenen Wirkstoffen enthält.

9. Implantatkörper nach einem der Ansprüche 1-8, wobei der Wirkstoff ein Gemisch aus Wachstumsfaktoren und einem Antibiotikum enthält.

10. Implantatkörper nach einem der Ansprüche 3-9, wobei der Füllwerkstoff resorbierbar oder nicht resorbierbar, organisch oder anorganisch ist und dämpfende oder versteifende Eigenschaften aufweist.

11. Implantatkörper nach einem der Ansprüche 3-10, wobei das Hohlraumsystem ganz oder teilweise mit dem Füllwerkstoff aufgefüllt oder beschichtet ist in der Weise, dass die innere Füllung sich auf das Innere des Implantatkörpers beschränkt oder diesen vollständig ausfüllt, jedoch dessen Oberfläche nicht überragt.

12. Implantatkörper nach einem der Ansprüche 3-10, wobei der Füllwerkstoff regelmäßig oder unregelmäßig aus den Poren der Oberfläche hervorquillt und tropfenförmige und/oder kugelförmige oder ähnlich konfigurierte Erhebungen über die Oberfläche bildet, die gleichmäßig oder ungleichmäßig über die Oberfläche verteilt sind.

13. Implantatkörper nach Anspruch 12, derart ausgebildet, dass der Füllwerkstoff Mikrobewegungen mehrerer gleicher oder ähnlicher Implantatkörper, beispielsweise in einer Schüttung, ganz oder nahezu ganz verhindert.

14. Implantatkörper nach einem der Ansprüche 3-13, wobei als Füllwerkstoff ein ein resorbierbares Polymer, beispielsweise eine Polyaminosäure, ein Polylactid, Polyglactin oder Glycosid, Polyacetale, Polysäureamide oder Polyester, oder ein nicht resorbierbares Polymer, wie Polymethylmethacrylate oder Agarose oder ein Gemisch aus Agarose und einem Calciumphosphat oder Calciumcarbonat verwandt wird.

15. Verfahren zur Herstellung eines Implantatkörpers nach einem der Ansprüche 1-14, mit den Schritten: Abfüllen einer Schüttung von unter Unterdruck expandierbaren formgebenden Elementen in einem Werkzeug mit einem die Struktur des Implantatkörpers bildenden Werkstoff, Beaufschlagen des Werkzeugs vor oder nach der Abfüllung mit einem definierten Unterdruck, danach Entfernen der formgegebenen Elemente, und mindestens teilweises Auffüllen oder Beschichten des Hohlraumsystems mit dem Wirkstoff.
